# EUROPÄISCHE PATENTSCHRIFT

(11) **EP 2 298 102 B1**
(45) Veröffentlichungstag und Bekanntmachung des Hinweises auf die Patenterteilung: **16.10.2013**
(21) Anmeldenummer: 09168688.1
(22) Anmeldetag: 26.08.2009
(51) Int. Cl.: A43B 7/22, A43B 7/14, A61F 5/14

(54) **Innensohle für Schuhe**
Inner sole for shoes
Semelle intérieure pour chaussures

(43) Veröffentlichungstag der Anmeldung: 23.03.2011
(73) Patentinhaber: Iseppi, Mario, 1227 Geneve (CH); Hansen, Christian Thagaard, 24944 Flensburg (DE)
(72) Erfinder: Hansen, Christian Thagaard, 24944, Flensburg (DE)
(74) Vertreter: Bittner, Thomas L.

(56) Entgegenhaltungen:
- US-A- 2 154 997
- US-A- 2 287 341
- US-A- 2 421 088
- US-A- 2 423 622
- US-A- 3 421 518
- US-A1- 2002 014 024
- US-B1- 6 510 626

## Beschreibung

Die Erfindung betrifft eine Innensohle für Schuhe, wobei die Innensohle jeweils eine flächige Rückseite in Richtung der Laufsohle des Schuhs, mit der die vorgeschlagene Innensohle in Kontakt gebracht werden soll, und eine vorderseitige kuppelförmige Ausformung aufweist.

Um eine allumfassende Würdigung der vorliegenden Erfindung zu ermöglichen, soll zunächst der geschlossene Bewegungszyklus eines gehenden Menschen intellektuell betrachtet werden. Dieser geschlossene Bewegungszyklus betrifft nicht nur den Fuß, sondern umfasst die gesamte untere Extremität. Der Fuß muss hierfür den Boden berühren. Wenn der Fuß den Boden berührt, wirkt sich jede Bewegung von Teilen dieses Fußes auf alle anderen Teile der entsprechenden Beins aus.

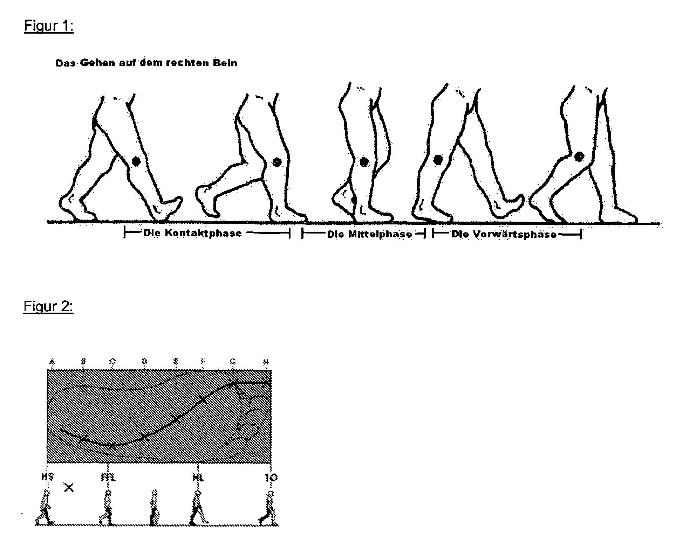

Die Gangbewegung eines jeden Beines wird in die Standphase und die Schwungphase unterteilt. Die Standphase wiederum ist in drei Komponentenphasen zu unterscheiden vgl. dazu Figur 1, die das Gehen des Menschen beispielhaft auf dem rechten Bein darstellt:
1. Die **Kontaktphase** als erste Komponentenphase der Standphase beginnt damit, dass der Fuß mit der Außenkante der Ferse auf den Boden trifft. Das Schienbein ist nach innen gedreht, und die Fuß-Innenseite ist leicht angehoben. Der Fuß rollt in der Phase weiter einwärts, bis die Mittefußknochen das volle Gewicht tragen. Das Schienenbein dreht sich wieder nach außen, und das Sprunggelenk proniert (inwärtskanten) bis zu 8°, wodurch sich der Fuß auf die Vorwärtsphase vorbereitet. Kurz gesagt, hat der Fuß den Stoß des Auftretens aufgenommen, sich an die unebene Fläche angepasst und abgeflacht. Die Kontaktphase endet mit dem vollen Bodenkontakt des Vorfußes. Die primäre Funktion dieser Phase ist es, den Stoß während des Auftretens zu absorbieren und sich an die unterschiedlichen Bodenbeläge anzupassen (Adaption).
2. Die **Mittelphase** als zweite Komponentenphase der Standphase beginnt mit dem vollen Bodenkontakt des Vorfußes und endet mit dem Abheben der Ferse von dem Boden. Das Körpergewicht läuft über den Fuß, wenn sich das Schienbein und der Rest des Körpers nach vorne bewegen. Die primäre Funktion des Fußes in dieser Phase ist es, die während der ersten Komponentenphase gewonnene Energie möglichst ohne Verlust zu speichern und aufzubewahren für die Vorwärtsphase - vergleichbar mit einem springenden Gummiball.
3. Die **Vorwärtsphase** als dritte Komponentenphase der Standphase beginnt mit dem Heben der Ferse, die Muskeln, Bänder und Sehnen werden angespannt. Der Vorderfuß und der Hinterfuß bilden zusammen ein Sprungbrett, mit dem die Zehen das Körpergewicht (vorwärts) vom Boden abheben können. Der Körper wird während dieser Komponentenphase nach vorne getrieben, wobei das Gewicht auf den anderen Fuß verlagert wird, wenn dieser andere Fuß Kontakt mit dem Boden erhält. Diese Phase dauert ca. 0,2 Sekunden und übernimmt 33% der gesamten Standphase. Während des Anfangs dieser dritten Komponentenphase der Standphase supiniert (Auswärtskanten) das Subtalargelenk und sorgt dafür, dass der Druckmittelpunkt unter der Vorderfußseite bleibt. Dies wiederum sorgt dafür, dass das Würfelbein sich mit dem Kahnbein verriegelt. Der Fuß verwandelt sich vom beweglichen Anpasser in einen starren Hebel, um den Körper während dieser Phase nach vorne zu treiben. Das Würfelbein ist aufgrund der Tatsache einzigartig, dass es der einzige Knochen im Fuß ist, der sowohl mit dem Mittelfußknochen-Gelenk (Tarsometatarsal- oder Lisfranc-Gelenk) und dem Fußwurzelknochen-Gelenk (Midtarsal- oder Chopart-Gelenk) artikuliert und ferner der einzige Knochen ist, der die Seitensäule mit dem quer verlaufenden Fußgewölbe verbindet. Folglich ist das Würfelbein der Schlussstein der steifen und statischen Seitensäule und gibt dem Fuß so eigene Stabilität. Das Verriegeln des Würfelbeins gegen das Kahnbein sorgt durch die beteiligten Bänder für ein sehr starken Halt und schont dabei die Muskeln, die sonst stark in Anspruch genommen würden, da die vertikalen Kräfte in diesem Moment 125% des Körpergewichts überschreiten können. Gegen Ende der Vorwärtsphase ist die Entriegelung des Würfelbeins erforderlich, nachdem die Verriegelung am Anfang der Vorwärtsphase geschah. Es kommt zur eine Co-Kontraktion des Musculus Fibularis Longus (auch Musculus Peroneus genannt) und des Musculus Tibialis Anterior, die zu Gegenkontraktionen führt und eine quer verlaufende Zieh- und Stützwirkung hervorruft, welche die Knochen des Mittelfußwurzelbereiches wesentlich ausrichtet. Die Stützwirkung der Sehne des Musculus Peroneus Longus um das Würfelbein ist wesentlich für die Kontrolle der Funktion des Quergewölbes für Stabilität und Anpassbarkeit. Um das Ende der Vorwärtsbewegung, in dem der große Zeh den Boden verlässt, zu erreichen, muss der Fuß jetzt nach innen rotieren - auch Pronation genannt. Würde das Würfelbein hier nicht freigegeben oder entriegelt werden, würde jedes Gelenk einen kleinen Teil seiner Bewegung und damit auch einen kleinen Teil seiner Kräfte verlieren, die zum Abrollen notwendig sind: es würde zu einer Hemmung der Muskelkraft, der Ausdauer, des Gleichgewichtes und der Tiefensensibilität kommen. Es gäbe dann außerdem eine Tendenz zu einer seitlichen Knöchelverstauchung, da diese Struktur im Grunde eine Erhebungsstruktur (Supination) ist und die Person keine funktionelle Einsenkung (Pronation) erreichen könnte. Der in Figur 2 dargestellte natürliche Kraftfluss durch den Fuß würde in einem solchen Fall unterbrochen oder eingeschränkt. Bevor der große Zeh den Boden verlässt, kommt es zu einer Dorsalflexion des großen Zehs zusammen mit den vier kleinen Zehen des gleichen Fußes und zur Plantarflexion des ersten Mittelfußknochen zusammen mit den anderen Mittelfußknochen des gleichen Fußes. Die Dorsalflexion des großen Zehs ist als Windlass-Effekt bekannt und wird aufgrund der Kontraktion des Musculus Extensor Hallicus Longus ermöglicht. Mit der Dorsalflexion des großen Zehs bewegen sich die Sesambeine nach vorne und oben um den Kopf des Mittelfußknochens und maximieren so die Spannung des Musculus Flexor Hallicus Longus.

Figur 1 soll das Gehen auf dem rechten Fuß wiedergeben und unterteilt dabei graphisch die Standphase in ihre drei Unterphasen: die Kontaktphase, die Mittelphase und die Vorwärtsphase.

Figur 2 soll den natürlichen Kraftfluss durch den Fuß näher erläutern. Der Kraftfluss beginnt leicht seitlich in der Ferse und fließt dann nach vorne zwischen den ersten und zweiten Mittelfußknochen und verlässt den Fuß durch den großen Zeh.

Zahlreiche Verfasser, die sich mit Gesundheitsproblemen insbesondere mit Problemen der unteren Extremität von Menschen beschäftigt haben, sind auf Grund ihrer durchgeführten Beobachtungen zur Überzeugung gekommen, dass die Füße von Menschen aus Naturvölkern, die keine Schuhe tragen, gerade solche Behinderungen nicht aufweisen, die bei Menschen, die stets und lange andauernd Schuhe tragen, oft anzutreffen sind: Hallux Valgus, Plantar Fasciitis, Ballenzehen, Hammerzehen und allgemein schmerzende Füße sind typische Beispiele solcher Behinderungen.

Kurz zusammengefasst kann festgehalten werden: In Gesellschaften, in denen keine Schuhe getragen werden, haben die Fußmuskeln Bewegungsfreiheit und die Gelenke bleiben geschmeidig. Deshalb findet man bei diesen Menschen nur äußerst selten Funktionsstörungen.

Bei Menschen, die Schuhe tragen, schränken die Schuhe für gewöhnlich durch ihre Art die natürliche Bewegungen des Fußes und die Abfolgen zur adaptiven Muskelaktivierung ein, die zur Stabilisierung der Fußstruktur vor und während der vollen Gewichtsbelastung und beim Abrollen erforderlich sind.

Um die vorstehend wiedergegebenen Gesundheitsprobleme zu lindern, wurden bereits Vorschläge für verschiedene rehabilitative Innensohlen gemacht. So schlägt beispielsweise die US 5,404,659 eine Innensohle für einen Schuh vor, bei der zur Anregung des Golgi Tendon Organs eine sehr weitflächige kuppelförmige Ausformung vorgesehen ist, die fast 50% der gesamten Innensohlenfläche ausmacht und den Fuß somit zu einer Gewölbe umschließenden, konkaven Zwangshaltung zwingt. Nachteilig an dieser bekannten Innensohle ist das Verhindern des in Absatz [0003] beschriebenen Windlass-Effeks und damit des letzten und sehr wichtigen Teils der Standphase: die Vorwärtsphase. Die bekannte Innensohle zielt mit ihrer kuppelförmigen Ausformung auf einen Bereich, der als Spitze des FußGewölbes definiert wird durch das laterale Keilbein, das Wurzelbein und das Kahnbein. Nach Ansicht des Erfinders ist jedoch für einen natürlichen Gang mit Schuhen die dynamische Vertiegelung/Entriegelung des Würfelbein von elementarer Wichtigkeit, was mit der bekannten Innensohle verhindert wird. Da der Peroneus Longus das Würfelbein umschlingt, ist es wichtig, dass das Würfelbein nachgibt. Andernfalls wird der Peroneus geschwächt. Es kann also die Erkenntnis des Erfinders hervorgehoben werden, dass es wichtig ist, nicht die Gewölbe umschließende Fußhaltung künstlich mit eine übermäßig großen kuppelförmigen Ausformung aufzubauen, sondern den Fuß dabei zu unterstützen, seine ihm von der Natur zugedachte Aufgabe optimal zu erfüllen. Darüber hinaus zeigte sich, dass das Tragen eines Schuhs mit der bekannten Innensohle eher als unangenehm empfunden.

Die auf sich als nächster Stand der Technik Bezug nehmenden europäischen Patentanmeldungen EP 1 041 947 und EP 1 423 062 versuchen letztendlich die durch die US 5,404,659 vorgeschlagene Innensohle zu optimieren, In beiden Fallen liegt jedoch eine sehr weitflächige kuppelförmige Ausformung mit den bereits genannten Nachteilen vor, in beiden Fällen soll das Golgi Tendon Organ angeregt werden mit einer kuppelförmige Ausformung, deren Zielpunkt durch den Gelenkpunkt des lateralen Keilbeins, des Würfelbeins und des Kahnbeins definiert ist.

Aus dem Dokument US 2 423 622 A ist eine Innensohle zur Korrektur von Fußabnormitäten bekannt. Der Oberbegriff des Anspruchs 1 ist aus diesem Dokument bekannt.

Im Dokument US 3 421 518 A wird die vollflächige Unterstützung der Würfelbeins mittels einer Innensohlenausformung vorgeschlagen, wobei eine solche Ausformung hier nicht zwangsläufig kuppelförmig ausgestaltet sein muss, sondern sich auch ausdehnen kann bis in den Randbereich der Innensohle.

Gegenstand der US 2002 014 024 A1 ist die Ausgestaltung einer sehr weitflächigen kuppelförmigen Ausformung, welche jedoch insbesondere gemäß Figur 2 auf eine Unterstützung des Kahnbeins zielt.

Das Dokument US 2 287 341 A, genauso aber auch das Dokument US 6 510 626 B1**,** schlagen die beidseitig seitliche Unterstützung des Fußes im Bereich von Kahn- und Würfelbein durch die Innensohle eines Schuhs vor.

Gegenstand des Dokumentes US 2 154 997A und auch des Dokumentes USA 2 421 088 A ist die vollflächige, eher außen liegende Unterstützung von Kahn- und Würfelbeins mittels einer die komplette Breite des Innenschuhs ausmachenden Erhöhung der Innensohle.

Nach intensiven Beobachtungen innerhalb der täglichen Praxis eines Chiropraktikers kam der Erfinder zu der Überzeugung, dass die oben gewürdigten Schriften allesamt von der Lösung des zugrunde liegenden Problems wegführen, wobei dieses Problem zusammengefasst werden kann durch die Zielsetzung, eine Innensohle für einen Schuh der Öffentlichkeit zur Verfügung zu stellen, die ein schmerz- und ermüdungsfreies, natürliches Gehen ermöglicht.

Die Aufgabe wird gelöst durch eine Innensohle nach Anspruch 1.

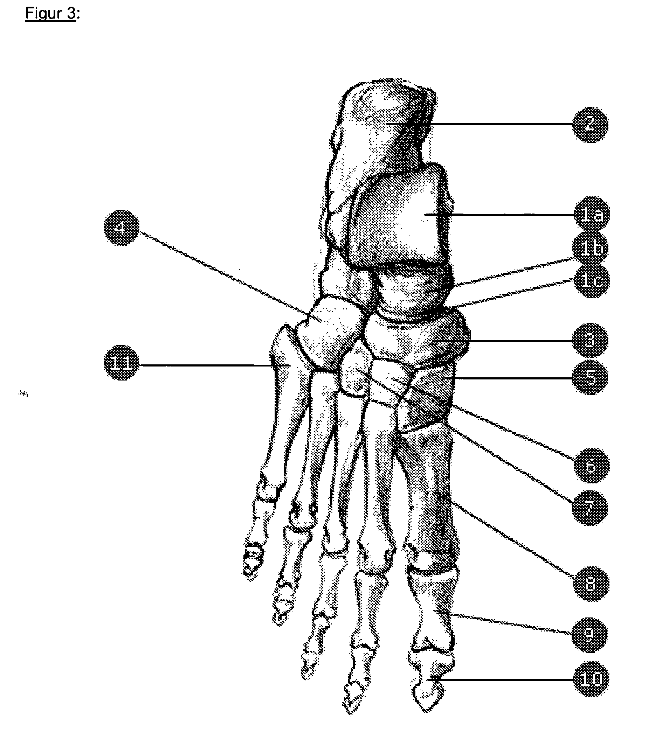

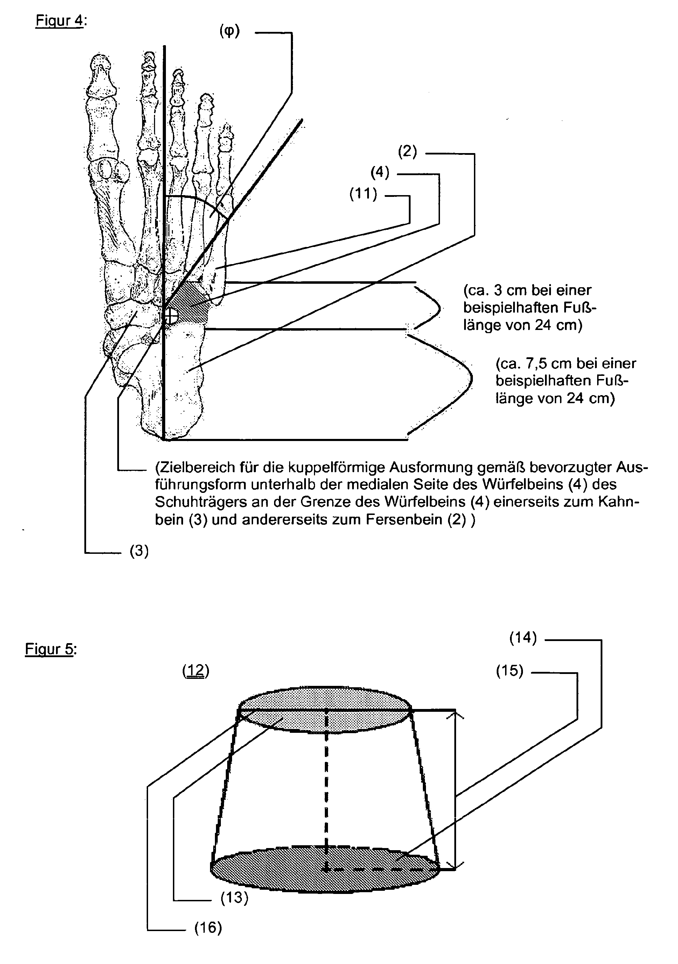

Die kuppelförmige Ausformung (12) der Innensohle ist unterhalb der medialen Seite des Würfelbeins (4) des Schuhträgers an der Grenze des Würfelbeins (4) einerseits zum Kahnbein (3) und andererseits zum Fersenbein (2) positioniert. In diesem Zusammenhang wird zum einen auf die Figur 3 hingewiesen, die den Knochenbau eines menschlichen Fußes zeigt und alle wesentlichen Knochen, die in dieser Schrift genannt werden, mit Namen benennt. Zum anderen wird auf die Figur 4 verwiesen, die genauso den menschlichen Fuß unter Nennung seiner für die Erfindung wesentlichen Knochen, sowie ferner den Zielpunkt der kuppelförmigen Ausformung entsprechend der hier vorliegenden Erfindung in einer seiner bevorzugten Ausführungen zeigt.

Die kuppelförmigen Ausformung (12) ist elastisch ausgeführt, wobei Ausführungsvariationen mit unterschiedlichen Härten als bevorzugt gelten. In zahlreichen Versuchen, die der vorliegenden Schrift zu Grunde liegen, zeigte es sich, dass die Basisfläche der kuppelförmigen Ausformung (12) bevorzugt sogar auf 10% der Innensohlenfläche, ganz besonders bevorzugt sogar auf eine Fläche in einem Bereich von weniger als 4 % bis 8% der Innensohlenfläche reduziert werden kann. In diesem Fall sollte jedoch der Träger eines solchen Schuhs das Laufen auf diesen erfindungsgemäßen Innensohlen mit kuppelförmigen Ausformung (12), die eine besonders stark reduzierte Basisfläche aufweisen, intensiv trainieren, da es sonst unter Umständen als weniger angenehm empfunden werden könnte.

Die kuppelförmigen Ausformung (12) ist gewöhnlich in Form eines basis- und spitzenseitig abgerundeten Kegel- bzw. Pyramidenstumpfes ausgebildet, wobei die Höhe (15) der kuppelförmigen Ausformung (12) bevorzugt in einem Bereich von 3 bis 20 mm liegt. Die dem Würfelbein (4) des Schuhträgers zugewandte abgerundete Spitze (13) des Kegel- bzw. Pyramidenstumpfes kann somit kreisrund bzw. quadratisch sein. In einer ganz besonders bevorzugten und seitens des Erfinders als die Beste angesehenen Ausführungsform weist der Kegel- bzw. Pyramidenstumpf zumindest an seiner dem Würfelbein (4) des Schuhträgers zugewandten abgerundeten Spitze (13) ein Rechteck bzw. ein Ellipse auf. Die kuppelförmige Ausformung weist ein Längs- / Querverhältnis in einem Bereich von 1,2 : 1 bis 3 : 1 auf.

Sofern der Kegel- bzw. Pyramidenstumpf an seiner dem Würfelbein (4) des Schuhträgers zugewandten abgerundeten Spitze (13) ein Rechteck bzw. ein Ellipse aufweist mit einem Längs-/Querverhältnis mindestens in einem Bereich in einem Bereich von 1 : 1 bis 4 : 1, kann der kuppelförmigen Ausformung (12) an ihrer Spitze (13) besonders leicht eine erfindungsgemäß vorgesehene Längsachse (16) zugeordnet werden. Weiterhin verläuft die Längsachse (16) der kuppelförmigen Ausformung (12) entlang der medialen Kante des Würfelbeins (4) und schließt somit mit der Längsachse der Innensohle einen Winkel (ϕ) von 5° bis 35°, ganz besonders bevorzugt einen Winkel (ϕ) von 25° bis 35° ein.

Zur Veranschaulichung der kuppelförmigen Ausformung (12) als Kegelstumpf wird insbesondere auf die Figur 5 verwiesen, die einen entsprechenden Kegelstumpf zeigt. Die Lage des Winkels (ϕ) wird insbesondere in der Figur 4 weiter veranschaulicht.

In einer ersten möglichen Ausführungsvariante der Innensohle ist diese Innensohle mit der kuppelförmigen Ausformung (12) unlöslich verbunden. Dieses kann umgesetzt sein, indem Innensohle und kuppelförmige Ausformung (12) separat gefertigt und anschließend unlöslich verklebt werden; genauso kann dieses umgesetzt sein, indem Innensohle und kuppelförmige Ausformung (12) einstockig aus einem geeigneten Kunststoffmaterial gegossen sind, ohne auf diese beiden Umsetzungsmöglichkeiten in irgendeiner Art beschränkt zu sein.

In einer zweiten möglichen Ausführungsvariante der Innensohle weisen sowohl die Innensohle wie auch die kuppelförmige Ausformung (12) Verbindungskomponenten auf, wobei die Verbindungskomponenten der Innensohle mit den Verbindungskomponenten der kuppelförmige Ausformung (12) so ausgebildet sind, dass Innensohle und kuppelförmige Ausformung (12) schwer lösbar miteinander verbunden sind. Diese Lösbarkeit ist dann gewünscht, wenn die vom Erfinder präferierte Möglichkeit eines Austausches der kuppelförmigen Ausformung (12) bei beibehaltender Innensohle gegeben sein soll. Bei einem ermöglichten Austausch der kuppelförmigen Ausformung (12) kann diese bei Verschleiß oder bei dem Wunsch nach einer anderen Härte besonders einfach und komfortabel ersetzt werden.

Die Verbindungskomponenten zwischen Innensohle und kuppelförmiger Ausformung (12) sind in diesem Fall bevorzugt ausgesucht aus der Liste, umfassend
■ Klettbänder,
■ eingelassene Nuten in der Innensohle und in die Nuten eingreifende Federn unterhalb der Basis (14) der kuppelförmigen Ausformung (12),
■ eingelassene Nuten in der Basis (14) der kuppelförmigen Ausformung (12) und in die Nuten eingreifende Federn an der Vorderseite der Innensohle.

In dem Fall, dass als Verbindungskomponenten zwischen Innensohle und kuppelförmiger Ausformung (12) eingelassene Nuten in der Innensohle gewählt werden, gilt es als bevorzugt, wenn diese eingelassenen Nuten in der Innensohle in einem Winkel von 80° bis 100° zur Längsachse (16) der kuppelförmigen Ausformung (12) verlaufen. Bei einer solchen Winkelwahl, bei denen die eingelassenen Nuten in der Innensohle und dazu korrespondierend die in die Nuten eingreifenden Federn unterhalb der Basis (14) der kuppelförmigen Ausformung (12) nahezu rechtwinkelig zur Längsachse (16) der kuppelförmigen Ausformung (12) verlaufen, sind Innensohle und kuppelförmiger Ausformung (12) besonders widerstandsfähig miteinander verbunden, weshalb sich eine solche Ausrichtung von Nuten und Federn insbesondere für Sportschuhe eignet. In einer ganz besonders bevorzugten Ausführungsvariante dieser beschriebenen Ausführungsform verlaufen die in der Innensohlen eingelassenen Nuten bis zu mindestens einem Außenrand der Innensohle, so dass die Federn unterhalb der Basis (14) der kuppelförmigen Ausformung (12) vom Außenrand der Innensohle aus in die eingelassenen Nuten der Innensohle eingeschoben werden können.

In dem Fall, dass als Verbindungskomponenten zwischen Innensohle und kuppelförmiger Ausformung (12) eingelassene Nuten in der Basis (14) der kuppelförmigen Ausformung (12) gewählt werden, gilt es als bevorzugt, wenn diese eingelassenen Nuten entlang der Längsachse (16) der kuppelförmigen Ausformung (12) verlaufen. Die zu den eingelassenen Nuten entlang der Längsachse (16) der kuppelförmigen Ausformung (12) korrespondierenden Federn sind auf der Vorderseite der Innensohle ausgebildet. Sofern die Nuten in der Basis (14) der kuppelförmigen Ausformung (12) bis zum äußeren Rand der kuppelförmigen Ausformung (12) geführt sind, ist ein Einschieben der Nuten vom Ende der Federn aus besonders einfach und bequem. Einrastelemente bei den Nuten und zugehörige Federn verhindern zum einen ein unbeabsichtigtes Verrutschen der kuppelförmigen Ausformung (12) gegenüber der Innensohle und erleichtern zum anderen ein exaktes Ausrichten der kuppelförmigen Ausformung (12) zur Innensohle.

Sofern die Verbindungskomponenten zwischen Innensohle und kuppelförmiger Ausformung (12) mittels Nuten und in die Nuten einzuführende Federn realisiert werden, gilt es als besonders bevorzugt, wenn die eingelassene Nuten hinterschnitten und die Federn dazu korrespondierend sich nach außen verbreiternd ausgebildet sind.

Es ist möglich, die Innensohle als integraler Bestandteil eines Schuhs auszubilden. In diesem Fall wird die Innensohle mit der Laufsohle eines Schuhs und gegebenenfalls zusätzlich mit dem Obermaterial dieses Schuhs verklebt und/oder vernäht.

Genauso ist es möglich und gilt als besonders bevorzugte Ausführungsform der vorliegenden Erfindung, wenn die Innensohle als Einlage für vorgegebene Schuhe ausgearbeitet ist. In diesem Fall ist es auch möglich, die Innensohle als so genannte ¾-Sohle anzufertigen und anzubieten, deren Fixierung auf einer vollständigen Innensohle innerhalb eines vorgegebenen Schuhs mittels doppelseitiger Klebebänder erfolgen kann. Dabei ist es üblich, solche ¾-Sohlen vorne und gegebenenfalls seitlich so weit zu kürzen, bis sie in den vorgegebenen Schuh hineinpassen.

### Begriffsliste:

(1) Sprungbein (Talus)
   (1a) Gelenkfläche des Sprungbeins
   (1b) Hals des Sprungbeins
   (1c) Kopf des Sprungbeins
(2) Fersenbein (Calcaneus)
(3) Kahnbein (Naviculare)
(4) Würfelbein (Cuboideum)
(5-7) Keilbeine (Cuneiforme I-III)
(8) größter Mittelfußknochen (Phalangen)
(11) kürzester Mittelfußknochen (Metatarsalia II)
   (ϕ) Winkel zwischen der Langsachse der kuppelförmigen Ausformung und der Längsachse der Innensohle
(12) kuppelförmige Ausformung
(13) Spitze der als Kegelstumpf ausgebildeten kuppelförmigen Ausformung
(14) Basis der als Kegelstumpf ausgebildeten kuppelförmigen Ausformung
(15) Höhe der kuppelförmigen Ausformung
(16) Längsachse an der Spitze der kuppelförmigen Ausformung

## Patentansprüche

1. Innensohle für einen Schuh, wobei
- die Innensohle eine flächige Rückseite in Richtung der Laufsohle des Schuhs und eine vorderseitige kuppelförmige Ausformung aufweist,
- die kuppelförmige Ausformung (12) eine Basisfläche von maximal 15 % der Innensohlenfläche aufweist,
- die kuppelförmige Ausformung (12) unterhalb des Würfelbeins (4) des Schuhträgers positioniert ist,
**dadurch gekennzeichnet, dass**
- die kuppelförmige Ausformung (12) unterhalb der medialen Seite des Würfelbeins (4) des Schuhträgers an der Grenze des Würfelbeins (4) einerseits zum Kahnbein (3) und andererseits zum Fersenbein (2) positioniert ist,
- die kuppelförmige Ausformung (12) ein Längs- /Querverhältnis in einem Bereich von 1,2 : 1 bis 3 : 1 aufweist und
- die Längsachse (16) der kuppelförmigen Ausformung (12) entlang der medialen Kante des Würfelbeins (4) verläuft und somit mit der Längsachse der Innensohle einen Winkel (ϕ) von 5° bis 35° einschließt.

2. Innensohle nach Patentanspruch 1, **dadurch gekennzeichnet, dass** die kuppelförmige Ausformung (12) eine Basisfläche von maximal 10 % der Innensohlenfläche aufweist.

3. Innensohle nach Patentanspruch 1 oder 2, **dadurch gekennzeichnet, dass** die kuppelförmige Ausformung (12) eine Höhe (15) in einem Bereich von 3 bis 20 mm aufweist.

4. Innensohle nach einem der Patentansprüche 1 bis 3, **dadurch gekennzeichnet, dass** die kuppelförmige Ausformung (12) unlöslich mit der Innensohle verbunden ist.

5. Innensohle nach einem der Patentansprüche 1 bis 3, **dadurch gekennzeichnet, dass** sowohl die Innensohle wie auch die kuppelförmige Ausformung (12) Verbindungskomponenten aufweisen, wobei die Verbindungskomponenten der Innensohle mit den Verbindungskomponenten der kuppelförmigen Ausformung (12) ausgebildet sind zur lösbaren Verbindung von Innensohle und kuppelförmiger Ausformung (12).

6. Innensohle nach Patentanspruch 5, **dadurch gekennzeichnet, dass** die Verbindungskomponenten ausgesucht aus der Liste, umfassend:
- Klettbänder,
- eingelassene Nuten in der Innensohle und in die Nuten eingreifende Federn unterhalb der Basis (14) der kuppelförmigen Ausformung (12),
- eingelassene Nuten in der Basis (14) der kuppelförmigen Ausformung (12) und in die Nuten eingreifende Federn an der Vorderseite der Innensohle.

7. Innensohle nach Patentanspruch 6, **dadurch gekennzeichnet, dass** die eingelassenen Nuten in der Innensohle in einem Winkel von 80° bis 100° zur Längsachse (16) der kuppelförmigen Ausformung (12) verlaufen.

8. Innensohle nach Patentanspruch 6, **dadurch gekennzeichnet, dass** die eingelassenen Nuten in der Basis (14) der kuppelförmigen Ausformung (12) entlang der Längsachse (16) der kuppelförmigen Ausformung (12) verlaufen.

9. Innensohle nach einem der Patentansprüche 6 bis 8, **dadurch gekennzeichnet, dass** die eingelassenen Nuten hinterschnitten und die Federn sich nach außen verbreiternd ausgebildet sind.

10. Innensohle nach einem der Patentansprüche 1 bis 9, **dadurch gekennzeichnet, dass** die Innensohle als Einlage für vorgegebene Schuhe ausgearbeitet ist.

11. Innensohle nach einem der Patentansprüche 1 bis 9, **dadurch gekennzeichnet, dass** die Innensohle integraler Bestandteil eines Schuhs ist.

## Claims

1. Insole for a shoe, wherein
- the insole has a flat back side in direction of the shoe outsole and a dome-shaped structure on the front side,
- the dome-shaped structure (12) has a base surface of a maximum of 15% of the insole surface,
- the dome-shaped structure (12) is positioned under the cuboid bone (4) of the shoe
wearer,
**characterized in that**
- the dome-shaped structure (12) is positioned under the medial side of the cuboid bone (4) of the shoe wearer where the cuboid bone (4) borders the navicular bone (3) on one side and the calcaneus bone (2) on the other side,
- the dome-shaped structure (12) has a longitudinal-transverse ratio in a range from 1,2:1 to 3: 1, and
- the longitudinal axis (16) of the dome-shaped structure (12) extends along the medial edge of the cuboid bone (4) and encloses an angle (ϕ) of 5° to 35° with the longitudinal axis of the insole.

2. Insole according to claim 1, **characterized in that** the dome-shaped structure (12) has a base surface of a maximum of 10% of the insole surface.

3. Insole according to claim 1 or 2, **characterized in that** the dome-shaped structure (12) has a height (15) in a range from 3 to 20 mm.

4. Insole according to one of claims 1 to 3, **characterized in that** the dome-shaped structure (12) is permanently connected to the insole.

5. Insole according to one of patent claims 1 to 3, **characterized in that** the insole and dome-shaped structure (12) both have connection components, wherein the connection components of the insole are formed with the connection components of the dome-shaped structure (12) for detachable connection of insole and dome-shaped structure (12).

6. Insole according to claim 5, **characterized in that** the connection components are selected from the list comprising:
- hook-and-loop strips,
- recessed grooves in the insole and springs engaging in the grooves under the base (14) of the dome-shaped structure (12),
- recessed grooves in the base (14) of the dome-shaped structure (12) and springs engaging in the grooves at the front side of the insole.

7. Insole according to claim 6, **characterized in that** the recessed grooves in the insole extend at an angle of 80° to 100° to the longitudinal axis (16) of the dome-shaped structure (12).

8. Insole according to claim 6, **characterized in that** the recessed grooves in the base (14) of the dome-shaped structure (12) extend along the longitudinal axis (16) of the dome-shaped structure (12).

9. Insole according to one of claims 6 to 8, **characterized in that** the recessed grooves are undercut and the springs are formed so as to widen outward.

10. Insole according to one of claims 1 to 9, **characterized in that** the insole is produced as an insert for certain shoes.

11. Insole according to one of claims 1 to 9, **characterized in that** the insole is an integral component part of the shoe.

## Revendications

1. Semelle intérieure pour une chaussure,
- la semelle intérieure comportant une partie arrière plane en direction de la semelle d'usure de la chaussure et un façonnage côté avant en forme de coupole,
- le façonnage en forme de coupole (12) comportant une surface de base d'un maximum de 15 % de la surface de la semelle intérieure,
- le façonnage en forme de coupole (12) étant positionné en-dessous de l'os cuboïde (4) du porteur de chaussure,
**caractérisée en ce que**
- le façonnage en forme de coupole (12) est positionné en-dessous du côté médial de l'os cuboïde (4) du porteur de chaussure, à la limite entre l'os cuboïde (4) et d'une part l'os scaphoïde (3) et d'autre part le calcanéum (2),
- le façonnage en forme de coupole (12) présente un rapport longueur à transversale de l'ordre de 1,2 : 1 à 3 : 1 et
- l'axe longitudinal (16) du façonnage en forme de coupole (12) s'étend le long de l'arête médiale de l'os cuboïde (4) et inclut de ce fait un angle (ϕ) de 5° à 35°.

2. Semelle intérieure selon la revendication 1, **caractérisée en ce que** le façonnage en forme de coupole (12) comporte une surface de base d'un maximum de 10 % de la surface de la semelle intérieure.

3. Semelle intérieure selon la revendication 1 ou la revendication 2, **caractérisée en ce que** le façonnage en forme de coupole (12) présente une hauteur (15) de l'ordre de 3 à 20 mm.

4. Semelle intérieure selon l'une quelconque des revendications 1 à 3, **caractérisée en ce que** le façonnage en forme de coupole (12) est relié de manière inamovible avec la semelle intérieure.

5. Semelle intérieure selon l'une quelconque des revendications 1 à 3, **caractérisée en ce qu'**aussi bien la semelle intérieure que le façonnage en forme de coupole (12) comportent des composants de liaison, les composants de liaison de la semelle intérieure étant formés avec les composants de liaison du façonnage en forme de coupole (12), pour la liaison amovible de la semelle intérieure et du façonnage en forme de coupole (12).

6. Semelle intérieure selon la revendication 5, **caractérisée en ce que** les composants de liaison sont sélectionnés dans la liste comprenant :
- des bandes auto-agrippantes,
- des rainures noyées dans la semelle intérieure et des ressorts s'engageant dans les rainures en-dessous de la base (14) du façonnage en forme de coupole (12),
- des rainures noyées dans la base (14) du façonnage en forme de coupole (12) et des ressorts s'engageant dans les rainures sur la face avant de la semelle intérieure.

7. Semelle intérieure selon la revendication 6, **caractérisée en ce que** les rainures noyées dans la semelle intérieure s'étendent sous un angle de 80° à 100° par rapport à l'axe longitudinal (16) du façonnage en forme de coupole (12).

8. Semelle intérieure selon la revendication 6, **caractérisée en ce que** les rainures noyées dans la base (14) du façonnage en forme de coupole (12) s'étendent le long de l'axe longitudinal (16) du façonnage en forme de coupole (12).

9. Semelle intérieure selon l'une quelconque des revendications 6 à 8, **caractérisée en ce que** les rainures noyées sont conçues avec des contre-dépouilles et les ressorts sont conçus en s'élargissant vers l'extérieur.

10. Semelle intérieure selon l'une quelconque des revendications 1 à 9, **caractérisée en ce que** la semelle intérieure est travaillée en tant que semelle orthopédique pour des chaussures prédéfinies.

11. Semelle intérieure selon l'une quelconque des revendications 1 à 9, **caractérisée en ce que** la semelle intérieure est une partie intégrante d'une chaussure.
